(19) Europäisches Patentamt
European Patent Office
Office européen des brevets

(11) EP 1 049 446 B1

(12) # FASCICULE DE BREVET EUROPEEN

(45) Date de publication et mention de la délivrance du brevet:
**21.04.2004 Bulletin 2004/17**

(21) Numéro de dépôt: **99938425.8**

(22) Date de dépôt: **16.08.1999**

(51) Int Cl.7: **A61K 7/06**, A61K 7/00

(86) Numéro de dépôt international:
**PCT/FR1999/001992**

(87) Numéro de publication internationale:
**WO 2000/012055 (09.03.2000 Gazette 2000/10)**

(54) **DISPOSITIF AEROSOL CONTENANT UN POLYCONDENSAT COMPRENANT AU MOINS UN MOTIF POLYURETHANNE ET/OU POLYUREE**

AEROSOLVORRICHTUNG, DIE EIN POLYKONDENSAT MIT MINDESTENS EINER POLYURETHANEINHEIT UND/ODER POLYHARNSTOFFEINHEIT ENTHÄLT.

AEROSOL DISPENSER CONTAINING A POLYCONDENSATE COMPRISING AT LEAST A POLYURETHANE AND/OR POLYUREA UNIT

(84) Etats contractants désignés:
**AT BE CH CY DE DK ES FI FR GB GR IE IT LI LU MC NL PT SE**

(30) Priorité: **27.08.1998 FR 9810780**

(43) Date de publication de la demande:
**08.11.2000 Bulletin 2000/45**

(73) Titulaire: **L'OREAL**
**75008 Paris (FR)**

(72) Inventeurs:
- **STURLA, Jean-Michel**
  **F-92100 Boulogne-Billancourt (FR)**
- **BREMENSON, Jean-Luc**
  **F-75019 Paris (FR)**
- **LE BOURHIS, François**
  **F-93300 Aubervilliers (FR)**
- **VILBERT, Arnaud**
  **36, Grande Rue, 91520 EGLY (FR)**

(74) Mandataire: **Bourdeau, Françoise et al**
**L'OREAL - D.I.P.I.**
**25-29 Quai Aulagnier**
**92600 Asnières (FR)**

(56) Documents cités:
**EP-A- 0 636 361**
**EP-A- 0 745 373**
**WO-A-97/25021**
**DE-A- 4 438 849**
**FR-A- 2 749 568**
**US-A- 5 626 840**

EP 1 049 446 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

L'invention a pour objet des dispositifs aérosols comprenant un récipient qui contient, dans un milieu cosmétiquement acceptable, un polymère multiséquencé comprenant au moins un motif polyuréthanne et/ou polyurée, ces dispositifs étant appropriés pour obtenir un débit initial en composition aérosol inférieur ou égal à 0,75 gramme par seconde. Elle vise également un procédé pour la mise en forme ou le maintien de la coiffure comprenant la mise en oeuvre de ces dispositifs ainsi que leur utilisation pour la fabrication de laques ou de sprays aérosol.

La fixation de la coiffure est un élément important du coiffage qui consiste à maintenir la mise en forme déjà réalisée ou à mettre en forme les cheveux et à les fixer simultanément.

Les produits capillaires pour la mise en forme et/ou le maintien de la coiffure les plus répandus sur le marché de la cosmétique sont des compositions à pulvériser essentiellement constituées d'une solution le plus souvent alcoolique ou aqueuse et d'un

ou plusieurs matériaux, généralement des résines polymères, dont la fonction est de former des soudures entre les cheveux, appelés encore matériaux fixants, en mélange avec divers adjuvants cosmétiques. Cette solution peut être conditionnée par exemple dans un récipient aérosol approprié mis sous pression à l'aide d'un propulseur.

Le conditionnement sous forme aérosol est spécialement pratique pour l'utilisateur qui obtient sans difficulté une répartition assez homogène au produit. Toutefois, ce type de conditionnement présente l'inconvénient de donner lieu à un dégagement de composes organiques volatiles (VOC) nocifs pour l'environnement. Ils proviennent notamment de la quantité de solvant organique mis en oeuvre et du gaz propulseur choisi pour fabriquer la composition On porte donc un interêt tout particulier à la réalisation de compositions cosmétiques conditionnées sous forme aérosol pour lesquelles la quantité de composés organiques volatiles rejetés est de plus en plus faible.

Les documents EP 636 361, WO97/2502, EP 745 373 et DE 44 38 849 décrivent des compositions cosmétiques comprenant des polymères à motifs uréthanes et/ou urée conditionnés en présence d'un gaz propulseur, éventuellement dans un dispositif aérosol. Ces documents parlent en toutes généralités des dispositifs aérosols, sans indiquer quelles caractéristiques mécaniques de ces dispositifs sont essentielles pour l'obtention d'une pulvérisation améliorée. Ces caractéristiques ne se trouvent pas plus dans FR 2 749 568, lequel n'est pas directement concerné par les polymères à motifs uréthanes et/ou urée.

La qualité de la pulvérisation obtenue au moyen d'un dispositif aérosol, c'est-à-dire essentiellement la distribution des gouttelettes dans l'espace à la sortie de la buse, dépend fortement de la constitution chimique de la composition mise en oeuvre. On porte donc un intérêt tout particulier à la réalisation de dispositifs aérosol qui donnent lieu à une qualité de pulvérisation optimale.

Il est connu par le brevet DE 195 41 326 de préparer des compositions de coiffage comprenant un polymère à motif polyuréthanne en tant que polymère fixant. Les dispositifs peuvent toutefois être améliorés en ce qui concerne notamment les propriétés cosmétiques qu'ils confèrent aux cheveux tout en offrant une meilleure qualité de pulvérisation.

De manière surprenante et inattendue, la Demanderesse a découvert, contre toute attente, qu'il était possible de réaliser des dispositifs aérosols qui satisfont aux exigences exprimées ci-dessus, en opérant une sélection d'une part, sur la composition cosmétique et d'autre part, sur les moyens de distribution de cette composition.

L'invention a pour objet un dispositif aérosol comprenant un récipient contenant une composition capillaire formée par un jus et au moins un propulseur ainsi que des moyens de distribution de la composition, caractérisé par le fait que:

(1) la composition comprend. dans un milieu cosmétiquement acceptable, au moins un polycondensat comprenant au moins une séquence polyuréthanne et/ou polyurée:

(2) le dispositif est approprié pour fournir un débit initial en composition aérosol inférieur ou égal à 0.75 gramme par seconde.

Un autre objet de l'invention concerne un procédé pour la mise en forme ou le maintien de la coiffure comprenant la mise en oeuvre de ce dispositif aérosol

Encore un autre objet de l'invention concerne l'utilisation de ce dispositif pour la fabrication d'une laque ou d'un spray aérosol.

Les polycondensats comprenant au moins une séquence polyuréthanne et/ou polyurée particulièrement visés par la présente invention sont ceux décrits dans les brevets EP 0 751 162, EP 0 637 600, FR 2 743 297 et EP 0 648 485 dont la Demanderesse est Titulaire, ainsi que le brevets EP 0 656 021 ou WO 94/03510 de la Société BASF et EP 0 619 111 de la Société National Starch.

Les polycondensats utilisés conformément à l'invention peuvent être solubles dans le milieu cosmétiquement acceptable, notamment après neutralisation par une base organique ou minérale, ou encore former une dispersion dans ce milieu. La dispersion peut comprendre alors au moins 0,05 % de tensioactif permettant la mise en dispersion

et le maintien en dispersion du polycondensat.

Selon l'invention, on peut utiliser tout type de tensioactif dans ladite dispersion, mais de préférence un tensioactif non ionique. La taille moyenne des particules du polycondensat dans la dispersion est de préférence comprise entre 0,1 et 1 micron.

A titre d'exemple, le polycondensat peut être formé par un arrangement de blocs, cet arrangement étant obtenu notamment à partir de:

(1) au moins un composé qui contient deux ou plus de deux atomes d'hydro gène actifs par molécule
(2) au moins un diol ou un mélange de diols contenant des radicaux acides ou leurs sels;
(3) au moins un di- ou polyisocyanate.

Avantageusement, les composés (1) sont choisis dans le groupe comprenant les diols, les diamines, les polyesterols, les polyétherols ou leur mélange.

Les composés (1) qui sont préférés sont les polyéthylène et les polypropylène glycols linéaires, en particulier ceux qui sont obtenus par réaction de l'oxyde d'éthylène ou de propylène avec l'eau ou du diéthylène ou du dipropylène glycol en présence d'hydroxyde de sodium en tant que catalyseur. Ces polyglycols ont généralement un poids moléculaire compris entre environ 600 et 20000.

D'autres composés organiques préférés sont ceux qui ont des groupes mercapto, amino, carboxyle ou hydroxyle. Parmi ceux-ci, on cite plus particulièrement les composés polyhydroxy tels que les polyéther diols, les polyester diols, les polyacétal diols, les polyamide diols, les polyester polyamide diols, les poly(alkylène éther) diols, les polythioéther diols et les polycarbonate diols.

Les polyéther diols préférés sont, par exemple, les produits de condensation d'oxyde d'éthylène, d'oxyde de propylène ou de tétrahydrofurane, leurs produits de copolymérisation ou de condensation, greffés ou blocs, tels que les mélanges de condensats d'oxyde d'éthylène et de propylène, et les produits de polymérisation d'oléfines, sous haute pression, avec les condensats d'oxyde d'alkylène. Des polyéthers appropriés sont par exemple préparés par condensation d'oxydes d'alkylène et d'alcools polyhydriques, tels que l'éthylène glycol, le 1,2-propylène glycol et le 1,4-butanediol.

Les polyesters diols, polyesters amides, polyamides diols sont de préférence saturés et sont obtenus, par exemple, à partir de la réaction d'acides polycarboxyliques saturés ou insaturés avec des alcools polyhydriques, des diamines ou des polyamines. Pour préparer ces composés, on peut utiliser, par exemple, l'acide adipique, l'acide succinique, l'acide phtalique, l'acide téréphtalique et l'acide maléique. Des alcools polyhydriques appropriés pour préparer les polyesters incluent par exemple l'éthylène glycol, le 1,2-propylène glycol, le 1,4-butanediol, le néopentyl glycol et l'hexane diol. On peut aussi utiliser des aminoalcools, par exemple l'éthanolamine. Des diamines appropriés pour préparer les amides polyesters sont l'éthylène diamine et l'hexaméthylène diamine.

Des polyacétals appropriés peuvent être préparés, par exemple, à partir de 1,4-butanediol ou d'hexanediol et de formaldéhyde. Des polythioéthers appropriés peuvent être préparés par exemple par réaction de condensation entre des thioglycol soit seuls ou en combinaison avec d'autres glycols tels que l'éthylène glycol, le 1,2-propylène glycol ou avec d'autres composés polyhydroxylés. Les composés polyhydroxylés contenant déjà des groupements urée ou uréthanne, des polyols naturels, qui peuvent être davantage modifiés, par exemple, l'huile de castor et les carbohydrates peuvent également être utilisés.

Plus préférentiellement, le composé du groupe (1) est un polyestérol, notamment un polyester diol formé par la réaction d'au moins un (di)-polyol ($1_a$) et d'au moins un acide ($1_b$). Le (di)- polyol ($1_a$) est en particulier choisi dans le groupe comprenant le néopentylglycol, le butanediol-1,4, l'hexanediol, l'éthylène-glycol, le diéthylène glycol, le propylèneglycol, le butylèneglycol, le néopentylglycol et (di)-polyéthylèneglycol. L'acide ($1_b$) est en particulier choisi dans le groupe comprenant l'acide phtalique, l'acide isophtalique, l'acide adipique et l'acide (poly)-lactique.

En tant que composé (2), on peut notamment utiliser un acide hydroxycarboxylique tel que l'acide diméthylol propanoïque (DMPA) ou un acide carboxylique 2,2-hydroxyméthyl. En général, le composé (2) est utile en tant que bloc de couplage. En tant que composés (2), on préfère ceux comprenant au moins un poly (acide-(alpha-hydroxycarboxyliquediol)).

Les composés (2) particulièrement préférés conformément à l'invention sont ceux choisis dans le groupe comprenant le 2,2-di-(hydroxyméthyl) acide acétique, le 2.2-dihydroxyméthyl acide propionique, le 2,2-dihydroxyméthyl acide butyrique, l'acide 2.2-dihydroxyméthyl acide pentanoïque.

Le di- ou polylsocyanate (3) peut être choisi en particulier oans le groupe comprenant l'hexaméthylène diisocyanate, l'isophorondiisocyanate (IDPI), le toluylendiisocyanate, le diphénylméthane 4,4'-diisocyanate (DPMD) et le dicyclohexylméthane 4,4'-diisocyanate (DCMD), le méthylène-di-p-phényl diisocyanate, le méthylène-bis(4-cyclohexylisocyanate), l'isophorone diisocyanate, le toluène diisocyanate, le 1,5-naphtalène diisocyanate, le 4,4'-diphénylméthane diisocyanate, le 2,2'-diméthyl-4,4'-diphénylméthane diisocyanate, le 1,3-phénylène diisocyanate, le 1,4-phénylène diisocyanate, des mélanges de 2,4- et de 2,6- toluène diisocyanate, le 2,2'-dichloro-4,4'-diisocyanato diphénylméthane, le 2,4-dibromo-1,5-diisocyanato naphtalène, le 1,4-diisocyanate butane, l'hexane-1,6-diisocyanate, le cyclohexane-1,4-diisocyanate.

Le polycondensat peut être formé à l'aide d'un composé supplémentaire (4) servant en général à allonger la chaîne du polycondensat. Ces composés (4) peuvent être choisis dans la groupe comprenant notamment les glycols saturés ou insaturés tel que l'éthylène glycol, le diéthylène glycol, le néopentylglycol, le triéthylène glycol, les aminoalcools tels que l'éthanolamine, la propanolamine, la butanolamine, les amines primaires hétérocyclique, aromatique, cycloaliphatique, et aliphatique, les diamines, les acides carboxylique tels que les acides carboxyliques aliphatique, aromatique, hétérocyclique comme l'acide oxalique, succinique, glutarique, adipique, sébacique, téréphtalique, les acides aminocarboxyliques. Les composés (4) préférés sont les diols aliphatiques.

Les polycondensats conformes à l'invention peuvent également être formés à partir de composés supplémentaires (5) ayant un squelette siliconé tels que les polysiloxanes, les polyalkylsiloxanes ou les polyarylsiloxanes notamment les polyéthylsiloxanes, les polyméthylsiloxanes et les polyphénylsiloxanes, comportant éventuellement des chaînes hydrocarbonées greffées sur les atomes de silicium.

Les séquences de polyuréthanne et/ou polyurée du polymère utilisées avantageusement présentent un motif répétitif de base répondant à la formule générale I' ci-après:

$$- X' - B - X' - CO - NH - R - NH - CO - \qquad (I')$$

dans laquelle

- X' représente O et/ou NH,
- B est un radical hydrocarboné bivalent, ce radical étant substitué ou non, et
- R est un radical divalent choisi parmi les radicaux alkylène de type aromatique, aliphatique en $C_1$ à $C_{20}$, cycloaliphatique en $C_1$ à $C_{20}$, ces radicaux étant substitués ou non.

De préférence, le radical B est un radical en $C_1$ à $C_{30}$ et est porteur d'un groupement présentant une ou des fonction(s) carboxylique(s) et/ou une ou des fonctions sulfoniques, lesdites fonctions carboxyliques et/ou sulfoniques étant sous forme libre ou bien neutralisées partiellement ou totalement par une base minérale ou organique.

Le radical R est avantageusement choisi parmi les radicaux répondant aux formules suivantes:

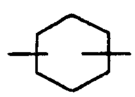 . 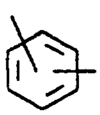 , 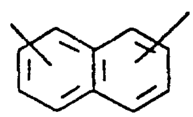

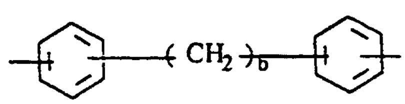

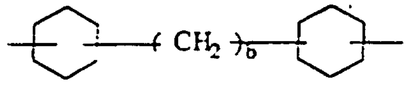

$$-(CH_2)_c-$$

dans lesquelles b est un nombre entier compris entre 0 et 3, et c un nombre entier compris entre 1 et 20, de préférence compris entre 2 et 12.

En particulier, le radical R est choisi parmi les radicaux héxaméthylène, 4,4'-biphénylèneméthane 2,4- et/ou 2,6-tolylène, 1,5-naphtylène, p-phénylène, méthylène- 4,4bis - cyclohexyle et le radical divaient dérivé de l'isophorone.

Le polycondensat mis en oeuvre conformément à l'invention comprenant au moins une séquence polyuréthanne et/ou polyurée peut avantageusement comprendre en outre au moins une séquence polysiloxane dont le motif répétitif de base répond par exemple à la formule générale (II') ci-après:

$$- X' - P - X' - CO - NH - R - NH - CO - \qquad (II'$$

dans laquelle :

- P est un segment polysiloxanique,
- X' représente O et/ou NH, et
- R est un radical divalent choisi parmi les radicaux alkylènes de type aromatique, aliphatique en $C_1$ à $C_{20}$, cycloaliphatique en $C_1$ à $C_{20}$, ces radicaux étant substitués ou non.

Avantageusement, le segment polysiloxanique P répond à la formule générale ci-après:

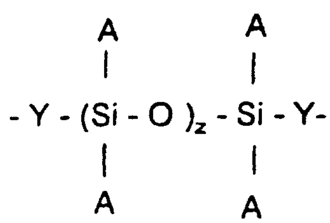

( III )

dans laquelle:

- les radicaux A, qui peuvent être identiques ou différents, sont choisis parmi d'une part les radicaux hydrocarbonés monovalents en $C_1$ à $C_{20}$ exempts ou substantiellement exempts d'insaturation éthylénique et, d'autre part, les radicaux aromatiques,
- Y représente un radical hydrocarboné bivalent, et
- z représente un nombre entier, choisi de telle sorte que le poids moléculaire moyen du segment polysiloxane soit compris entre 300 et 10 000.

En général, le radical bivalent Y est choisi parmi les radicaux alkylène de formule $-(CH_2)_a-$ , dans laquelle a représente un nombre entier pouvant être compris entre 1 et 10.

Les radicaux A peuvent être choisis parmi les radicaux alkyles, en particulier les radicaux méthyle, éthyle, propyle, isopropyle, butyle, pentyle, hexyle, octyle, décyle, dodécyle et octadécyle, les radicaux cycloalkyle, en particulier le radical cyclohexyle, les radicaux aryle, notamment phényle et naphtyle, les radicaux arylalkyle, notamment benzyle et phényléthyle, ainsi que les radicaux tolyle et xylyle.

Avantageusement, le jus comprend un solvant organique et le rapport pondérai du gaz propulseur au solvant organique est supérieur ou égal à 1,5, et de préférence supérieur ou égal à 1,75.

Le moyen de distribution, qui forme une partie du dispositif aérosol, est généralement constitué par une valve de distribution commandée par une tête de distribution, elle même comprenant une buse par laquelle la composition aérosol est vaporisée.

Conformément à l'invention, le débit initial en composition aérosol est mesuré pour une température voisine de 20°C à l'intérieur du dispositif aérosol. En pratique, on place le dispositif aérosol à la température ambiante et on mesure le débit initial en composition aérosol lorsque l'équilibre thermodynamique est atteint.

Le débit initial est la quantité moyenne de produit sortant sur cinquante secondes du dispositif aérosol non préalablement utilisé. Il est exprime en gramme par seconde.

Le débit initial de la composition aérosol ($D_{CA}$) correspond à la quantité de composition aérosol (jus + propulseur) sortant par unité de temps du dispositif aérosol non préalablement utilisé. Il est exprimé en mg/s et est mesuré

par la différence entre le poids de l'aérosol avant ($M_0$) et après ($M_1$) 10 secondes de vaporisation:

$$D_{CA} = (M_0 - M_1) / 10.$$

De manière avantageuse, le dispositif aérosol selon l'invention est approprié pour obtenir un débit initial en composition aérosol inférieur ou égal à 0,7 gramme par seconde.

Le débit initial en composition aérosol des dispositifs selon l'invention dépend d'une part de la composition, et d'autre part du moyen de distribution, les deux devant être appropriés pour obtenir les caractéristiques recherchées.

Les caractéristiques particulières définies ci-dessus peuvent être obtenues en sélectionnant les moyens de distribution appropriés et/ou en agissant sur la formulation.

Les valves appropriées pour les compositions particulières ci-dessus sont notamment des valves possédant un orifice de restriction interne de 0,33mm sans orifice de prise de gaz additionnelle et ayant un orifice de gicleur mesurant entre 0,33 et 0,51 mm. Avantageusement, on utilise un bouton poussoir possédant une buse tourbillonnaire avec un orifice de buse de dimension comprise entre 0,4 et 0,5 mm.

Les polycondensats utilisés conformément à l'invention peuvent être solubles dans le milieu cosmétiquement acceptable ou encore former une dispersion dans ce milieu. La dispersion peut comprendre alors au moins 0.05 % de tensio-actif permettant la mise en dispersion et le maintien en dispersion du polycondensat

Selon l'invention, on peut utiliser tout type de tensioactif dans ladite dispersion, mais de préférence un tensioactif non ionique. La taille moyenne des particules du polycondensat dans la dispersion est de préférence comprise entre 0,1 et 1 micron.

La composition conforme à l'invention comprend avantageusement, en proportion relative en poids par rapport au poids total de la composition, entre 0,1 et 20 % du polycondensat comprenant au moins une séquence polyuréthanne et/ou polyurée, et plus avantageusement encore entre 1 et 15 %, et encore plus avantageusement entre 2 et 8 % de ce polycondensat.

Elle comprend avantageusement entre 7,5 et 70 % d'un solvant organique, et plus avantageusement encore entre 10 et 50 %, et encore plus préférentiellement entre 10 et 25 % de ce polycondensat, en proportion relative en poids par rapport au poids total de la composition.

Conformément à l'invention le solvant organique est notamment choisi dans le groupe comprenant les alcools en $C_1$ à $C_4$ tels que l'éthanol ou l'isopropanol, l'acétone, la méthyléthylcétone, l'acétate de méthyle, l'acétate de butyle, l'acétate d'éthyle, le diméthoxyéthane, le diéthoxyéthane et leurs mélanges. De manière préférentielle, on utilise l'éthanol.

La proportion relative en poids, par rapport au poids total de la composition, en gaz propulseur dans la composition est avantageusement comprise entre 15 et 85 %, et plus avantageusement encore entre 25 et 60 %, et encore plus avantageusement entre 30 et 50 %.

Conformément à l'invention, on utilise, de préférence, comme gaz propulseur, un gaz soluble ou non dans la composition tel que le diméthyl éther, les hygrocarbures fluorés ou non, les gaz liquéfiés usuels ou un mélange de ces gaz propulseurs. De préférence, on utilise le diméthyl éther.

Les compositions conformes à l'invention peuvent par ailleurs contenir des additifs cosmétiques conventionnels choisis notamment parmi les corps gras, les agents épaississants, les adoucissants, les agents anti-mousse, les agents hydratants, les agents antiperspirants, les agents alcalinisants, les colorants, les pigments, les parfums, les conservateurs, les tensioactifs, les polymères hydrocarbonés, les silicones volatiles ou non, notamment les silicones anioniques, les polyols, les protéines et les vitamines.

En particulier, il peut être avantageux d'ajouter à la composition d'autres polymères fixants tels que des polymères fixants non ioniques, anioniques, cationiques ou amphotères.

L'invention pourra être mieux comprise à l'aide de l'exemple non limitatif ci-après.

Exemple:

On réalise un dispositif conforme à l'invention contenant la composition ci-après.

- Polycondensat polyester acide lactique / éthylène glycol P (MIS - EG) - acide diméthylol propanoïque (DMPA) - isophoronediisocyanate 4 %
- Aminométhyl propanol qs neutralisation
- Ethanol. 15 %
- Diméthyléther 35 %
- Eau déminéralisée qsp 100 %

On a utilisé une valve comprenant:

- un orifice de gicleur de 0.33 mm,
- pas d'orifice de prise de gaz additionnelle,
- un orifice de restriction interne de 0.33 mm

On a utilisé un bouton poussoir possédant une buse tourbillonnaire d'orifice de sortie de 0,45 mm.
Le débit initial mesuré à 20°C est de 0,65 gramme par seconde.

## Revendications

1. Dispositif aérosol comprenant un récipient contenant une composition capillaire formée par un jus et au moins un propulseur ainsi que des moyens de distribution de la composition, **caractérisé par le fait que**:

   (i) la composition comprend, dans un milieu cosmétiquement acceptable, au moins un polycondensat comprenant au moins une séquence polyuréthanne et/ou polyurée; et
   (ii) le dispositif est approprié pour fournir un débit initial en composition aérosol inférieur ou égal à 0,75 gramme par seconde,

   et le dispositif comprend une valve présentant un orifice de restriction interne de 0,33 mm sans orifice de prise de gaz additionnelle et ayant un orifice de gicleur mesurant entre 0,33 et 0,51 mm.

2. Dispositif selon la revendication 1, **caractérisé par le fait que** le débit initial en composition aérosol est inférieur ou égal à 0,7 gramme par seconde.

3. Dispositif selon l'une quelconque des revendications précédentes, **caractérisé par le fait que** le jus contient un solvant organique, le rapport pondéral du propulseur au solvant organique étant supérieur ou égal à 1,5, et de préférence supérieur ou égal à 1,75.

4. Dispositif selon l'une quelconque des revendications précédentes, **caractérisé par le fait que** le polycondensat est formé par un arrangement de blocs.

5. Dispositif selon la revendication précédente 4, **caractérisé par le fait que** l' arrangement de blocs est obtenu à partir de:

   (1) au moins un composé qui contient deux ou plus de deux atomes d'hydro gène actifs par molécule;
   (2) au moins un diol ou un mélange de diols contenant des radicaux acides ou leurs sels;
   (3) au moins un di- ou polylsocyanate.

6. Dispositif selon la revendication 5, **caractérisé par le fait que** les composés (1) sont choisis dans le groupe comprenant les diols, les diamines, les polyesterols, les polyétherols ou leur mélange.

7. Dispositif selon la revendication 5, **caractérisé par le fait que** le composé (2) est un acide carboxylique 2,2-hydroxyméthyl.

8. Dispositif selon la revendication 5, **caractérisé par le fait que** le composé (3) est choisi dans la groupe comprenant l'hexaméthylène diisocyanate, l'isophorondiiso-cyanate, le toluylendiisocyanate, le diphénylméthane 4,4'-diisocyanate, le dicyclohexylméthane 4,4'-diisocyanate, le méthylène-di-p-phényl diisocyanate, le méthylène-bis(4-cyclohexylisocyanate), l'isophorone diisocyanate, le toluène diisocyanate, le 1,5-naphtalène diisocyanate, le 4,4'-diphénylméthane diisocyanate, le 2,2'-diméthyl-4,4'-diphénylméthane diisocyanate, le 1,3-phénylène diisocyanate, le 1,4-phénylène diisocyanate, des mélanges de 2,4- et de 2,6- toluène diisocyanate, le 2,2'-dichloro-4,4'-diisocyanato diphénylméthane, le 2,4-dibromo-1,5-diisocyanato naphtalène, le 1,4-diisocyanate butane, l'hexane-1,6-diisocyanate et le cyclohexane-1,4-diisocyanate.

9. Dispositif selon la revendication 5, **caractérisé par le fait que** le polycondensat est formé à partir d'au moins un composé supplémentaire ayant un squelette siliconé et choisi dans le groupe comprenant les polysiloxanes, les polyalkylsiloxanes ou les polyarylsiloxanes.

**10.** Dispositif selon la revendication 9, **caractérisé par le fait que** les polyalkylsiloxanes ou les polyarylsiloxanes sont les polyéthylsiloxanes, les polyméthylsiloxanes et les polyphénylsiloxanes, comportant éventuellement des chaînes hydrocarbonées greffées sur les atomes de silicium.

**11.** Dispositif selon l'une quelconque des revendications1 à 3, **caractérisé par le fait que** les séquences de polyuréthanne et/ou polyurée du polymère présentent un motif répétitif de base répondant à la formule générale I' ci-après:

$$- X' - B - X' - CO - NH - R - NH - CO - \qquad (I')$$

dans laquelle :

- X' représente O et/ou NH,
- B est un radical hydrocarboné, ce radical étant substitué ou non, et
- R est un radical divalent choisi parmi les radicaux alkylène de type aromatique, aliphatique en $C_1$ à $C_{20}$, cycloaliphatique en $C_1$ à $C_{20}$, ces radicaux étant substitués ou non.

**12.** Dispositif selon la revendication 11, **caractérisé par le fait que** B est un radical hydrocarboné bivalent en $C_1$ à $C_{30}$.

**13.** Dispositif selon la revendication 11, **caractérisé par le fait que** le radical R est choisi dans le groupe comprenant les radicaux hexaméthylène, 4,4'-biphénylèneméthane, 2,4- et/ou 2,6-tolylène, 1,5-naphtylène, p-phénylène, méthylène- 4,4bis - cyclohéxyle et le radical divalent dérivé de l'isophorone.

**14.** Dispositif selon l'une quelconque des revendications 1 à 3, **caractérisé par le fait que** le polycondensat présente un motif répétitif de base répondant à la formule (II'):

$$- X' - P - X' - CO - NH - R - NH - CO - \qquad (II')$$

dans laquelle :

- P est un segment polysiloxanique,
- X' représente O et/ou NH, et
- R est un radical divalent choisi parmi les radicaux alkylène de type aromatique, aliphatique en $C_1$ à $C_{20}$, cycloaliphatique en $C_1$ à $C_{20}$, ces radicaux étant substitués ou non.

**15.** Dispositif selon l'une quelconque des revendications précédentes, **caractérisé par le fait que** la composition comprend, en proportion relative en poids, entre 0,1 et 20 % du polycondensat.

**16.** Dispositif selon la revendication précédente 15, **caractérisé par le fait que** la composition comprend, en proportion relative en poids, entre 1 et 15 % du polycondensat.

**17.** Dispositif selon la revendication précédente 15 ou 16, **caractérisé par le fait que** la composition comprend, en proportion relative en poids, entre 2 et 8 % du polycondensat.

**18.** Dispositif selon la revendication 3, **caractérisé par le fait que** la composition comprend un solvant organique, celui-ci étant présent à une concentration relative en poids comprise entre 7,5 et 70 %.

**19.** Dispositif selon la revendication 18, **caractérisé par le fait que** la composition comprend un solvant organique, celui-ci étant présent à une concentration relative en poids comprise entre tre 10 et 50 %.

**20.** Dispositif selon la revendication 18 ou 19, **caractérisé par le fait que** la composition comprend un solvant organique, celui-ci étant présent à une concentration relative en poids comprise entre 10 et 25 %.

**21.** Dispositif selon l'une quelconque des revendications précédentes, **caractérisé par le fait que** le gaz propulseur est présent à une concentration relative en poids comprise entre 15 et 70 %.

**22.** Dispositif selon la revendication 21, **caractérisé par le fait que** le gaz propulseur est présent à une concentration

relative en poids comprise entre 25 et 60 %.

**23.** Dispositif selon l'une quelconque des revendications précédentes 21 ou 22, **caractérisé par le fait que** le gaz propulseur est présent à une concentration relative en poids comprise entre 30 et 50 %.

**24.** Dispositif selon l'une quelconque des revendications précédentes, **caractérisé par le fait qu'**il comprend un bouton poussoir possédant une buse tourbillonnairé, la dimension de l'orifice de buse étant comprise entre 0,4 et 0,5 mm.

**25.** Dispositif selon l'une quelconque des revendications précédentes, **caractérisé par le fait que** la composition contient en outre des additifs cosmétiques conventionnels choisis dans le groupe comprenant les corps gras, les agents épaississants, les adoucissants, les agents anti-mousse, les agents hydratants, les agents antiperspirants, les agents alcalinisants, les colorants, les pigments, les parfums, les conservateurs, les tensioactifs, les polymères hydrocarbonés, les silicones volatiles ou non, notamment les silicones anioniques, les polyols , les protéines et les vitamines.

**26.** Dispositif selon l'une quelconque des revendications précédentes, **caractérisé par le fait que** la composition contient au moins un polymère fixant additionnel choisi dans le groupe comprenant les polymères fixants non ioniques, cationiques, anioniques ou amphotères.

**27.** Procédé capillaire pour la mise en forme ou le maintien de la coiffure, **caractérisé par le fait qu'**il comprend la mise en oeuvre d'un dispositif conforme à l'une quelconque des revendications 1 à 26.

**28.** Utilisation d'un dispositif selon l'une quelconque des revendications 1 à 26 pour la fabrication d'une laque ou d'un spray aérosol.

**Patentansprüche**

**1.** Aerosolvorrichtung, die einen Behälter mit einer Zusammensetzung für die Haarbehandlung, die aus einer flüssigen Phase und mindestens einem Treibmittel besteht, sowie Einrichtungen zur Verteilung der Zusammensetzung enthält, **dadurch gekennzeichnet, dass**:

(i) die Zusammensetzung in einem kosmetisch akzeptablen Medium mindestens ein Polykondensat enthält, das mindestens eine Polyurethansequenz und/oder Polyharnstoffsequenz aufweist, und
(ii) die Vorrichtung dazu geeignet ist, einen anfänglichen Durchsatz der Aerosolzusammensetzung von 0,75 Gramm pro Sekunde oder darunter zu liefern, und

die Vorrichtung ein Ventil enthält, das eine innere Verengung von 0,33 mm ohne Öffnung für eine zusätzliche Gaszufuhr aufweist und eine Düsenöffnung im Bereich von 0,33 bis 0,51 mm besitzt.

**2.** Vorrichtung nach Anspruch 1, **dadurch gekennzeichnet, dass** der anfängliche Durchsatz der Aerosolzusammensetzung höchstens 0,7 Gramm pro Sekunde beträgt.

**3.** Vorrichtung nach einem der vorhergehenden Ansprüche, **dadurch gekennzeichnet, dass** die flüssige Phase ein organisches Lösungsmittel enthält, wobei das Gewichtsverhältnis des Treibmittels und des organischen Lösungsmittels mindestens 1,5 und vorzugsweise mindestens 1,75 beträgt.

**4.** Vorrichtung nach einem der vorhergehenden Ansprüche, **dadurch gekennzeichnet, dass** das Polykondensat aus einer Anordnung von Blöcken besteht.

**5.** Vorrichtung nach Anspruch 4, **dadurch gekennzeichnet, dass** die Blockanordnung erhalten wird, ausgehend von:

(1) mindestens einer Verbindung, die zwei oder mehr als zwei aktive Wasserstoffatome pro Molekül enthält;
(2) mindestens einem Diol oder einem Gemisch von Diolen, die Säuregruppen oder deren Salze enthalten;
(3) mindestens einem Di- oder Polyisocyanat.

**6.** Vorrichtung nach Anspruch 5, **dadurch gekennzeichnet, dass** die Verbindungen (1) unter den Diolen, Diaminen,

Polyesterolen, Polyetherolen oder deren Gemischen ausgewählt sind.

7. Vorrichtung nach Anspruch 5, **dadurch gekennzeichnet, dass** die Verbindung (2) eine 2,2-Hydroxymethylcarbonsäure ist.

8. Vorrichtung nach Anspruch 5, **dadurch gekennzeichnet, dass** die Verbindung (3) unter Hexamethylendiisocyanat, Isophorondiisocyanat, Toluylendiisocyanat, Diphenylmethan-4,4'-diisocyanat, Dicyclohexylmethan-4,4'-diisocyanat, Methylen-di-pphenyldiisocyanat, Methylen-bis(4-cyclohexylisocyanat), Isophorondiisocyanat, Toluoldiisocyanat, 1,5-Naphthalindiisocyanat, 4,4'-Diphenylmethan-diisocyanat, 2,2'-Dimethyl-4,4'-diphenylmethandiisocyanat, 1,3-Phenylendiisocyanat, 1,4-Phenylendiisocyanat, Gemischen von 2,4- und 2,6-Toluoldiisocyanat, 2,2'-Dichlor-4,4'-diisocyanato-diphenylmethan, 2,4-Dibrom-1,5-diisocyanatonaphthalin, 1,4-Diisocyanatobutan, Hexan-1,6-diisocyanat und Cyclohexan-1,4-diisocyanat ausgewählt ist.

9. Vorrichtung nach Anspruch 5, **dadurch gekennzeichnet, dass** das Polykondensat ausgehend von mindestens einer zusätzlichen Verbindung gebildet wird, das ein Silicongerüst aufweist und unter den Polysiloxanen, Polyalkylsiloxanen oder Polyarylsiloxanen ausgewählt ist.

10. Vorrichtung nach Anspruch 9, **dadurch gekennzeichnet, dass** es sich bei den Polyalkylsiloxanen oder Polyarylsiloxanen um Polyethylsiloxane, Polymethylsiloxane und Polyphenylsiloxane handelt, die gegebenenfalls auf die Siliciumatome gepfropfte Kohlenwasserstoffketten enthalten.

11. Vorrichtung nach einem der Ansprüche 1 bis 3, **dadurch gekennzeichnet, dass** die Polyurethansequenzen und/oder Polyharnstoffsequenzen des Polymers eine wiederkehrende Grundeinheit der folgenden allgemeinen Formel I' aufweisen:

$$- X' - B - X' - CO - NH - R - NH - CO - \qquad (I'),$$

worin bedeuten:

- X' O und/oder NH,
- B eine Kohlenwasserstoffgruppe, wobei die Gruppe gegebenenfalls substituiert ist, und
- R eine zweiwertige Gruppe, die unter den Alkylengruppen vom aromatischen Typ, aliphatischen Typ mit 1 bis 20 Kohlenstoffatomen und cycloaliphatischen Typ mit 1 bis 20 Kohlenstoffatomen ausgewählt ist, wobei die Gruppen substituiert oder unsubstituiert vorliegen.

12. Vorrichtung nach Anspruch 11, **dadurch gekennzeichnet, dass** die Gruppe B eine zweiwertige Kohlenwasserstoffgruppe mit 1 bis 30 Kohlenstoffatomen ist.

13. Vorrichtung nach Anspruch 11, **dadurch gekennzeichnet, dass** die Gruppe R unter Hexamethylen, 4,4'-Biphenylenmethan, 2,4-und/oder 2,6-Tolylen, 1,5-Naphthylen, p-Phenylen, Methylen-4,4-bis-cyclohexyl und der von Isophoron abgeleiteten zweiwertigen Gruppe ausgewählt ist.

14. Vorrichtung nach einem der Ansprüche 1 bis 3, **dadurch gekennzeichnet, dass** das Polykondensat eine wiederkehrende Grundeinheit der folgenden Formel (II') aufweist:

$$- X' - P - X' - CO - NH - R - NH - CO \qquad (II'),$$

worin bedeuten:

- P ein Polysiloxansegment,
- X' O und/oder NH,
- R eine zweiwertige Gruppe, die unter den Alkylengruppen vom aromatischen Typ, aliphatischen Typ mit 1 bis 20 Kohlenstoffatomen und cycloaliphatischen Typ mit 1 bis 20 Kohlenstoffatomen ausgewählt ist, wobei die Gruppen substituiert oder unsubstituiert vorliegen.

15. Vorrichtung nach einem der vorhergehenden Ansprüche, **dadurch gekennzeichnet, dass** die Zusammensetzung

das Polykondensat in einer relativen Gewichtsmenge von 0,1 bis 20 % enthält.

**16.** Vorrichtung nach Anspruch 15, **dadurch gekennzeichnet, dass** die Zusammensetzung das Polykondensat in einer relativen Gewichtsmenge von 1 bis 15 % enthält.

**17.** Vorrichtung nach dem vorhergehenden Anspruch 15 oder 16, **dadurch gekennzeichnet, dass** die Zusammensetzung das Polykondensat in einer relativen Gewichtsmenge von 2 bis 8 % enthält.

**18.** Vorrichtung nach Anspruch 3, **dadurch gekennzeichnet, dass** die Zusammensetzung ein organisches Lösungsmittel enthält, das in einer relativen, auf das Gewicht bezogenen Konzentration von 7,5 bis 70 % vorliegt.

**19.** Vorrichtung nach Anspruch 18, **dadurch gekennzeichnet, dass** die Zusammensetzung ein organisches Lösungsmittel enthält, das in einer relativen Konzentration von 10 bis 50 Gew.-% enthalten ist.

**20.** Vorrichtung nach Anspruch 18 oder 19, **dadurch gekennzeichnet, dass** die Zusammensetzung ein organisches Lösungsmittel enthält, das in einer relativen Konzentration von 10 bis 25 Gew.-% enthalten ist.

**21.** Vorrichtung nach einem der vorhergehenden Ansprüche, **dadurch gekennzeichnet, dass** das Treibgas in einer relativen Konzentration von 15 bis 70 Gew.-% vorliegt.

**22.** Vorrichtung nach Anspruch 21, **dadurch gekennzeichnet, dass** das Treibgas in einer relativen Konzentration von 25 bis 60 Gew.-% vorliegt.

**23.** Vorrichtung nach einem der vorhergehenden Ansprüche 21 oder 22, **dadurch gekennzeichnet, dass** das Treibgas in einer relativen Konzentration von 30 bis 50 Gew.-% vorliegt.

**24.** Vorrichtung nach einem der vorhergehenden Ansprüche, **dadurch gekennzeichnet, dass** sie einen Betätigungsknopf mit einer Wirbeldüse aufweist, wobei die Abmessung der Düsenöffnung im Bereich von 0,4 bis 0,5 mm liegt.

**25.** Vorrichtung nach einem der vorhergehenden Ansprüche, **dadurch gekennzeichnet, dass** die Zusammensetzung ferner herkömmliche kosmetische Zusatzstoffe enthält, die unter den Fettsubstanzen, Verdickungsmitteln, beruhigenden Stoffen, Schaumverhütungsmitteln, Hydratisierungsmitteln, Antiperspirantien, Alkalisierungsmitteln, Farbmitteln, Pigmenten, Parfums, Konservierungsmitteln, grenzflächenaktiven Stoffen, Kohlenwasserstoff-haltigen Polymeren, flüchtigen oder nichtflüchtigen Siliconen und insbesondere anionischen Siliconen, Polyolen, Proteinen und Vitaminen ausgewählt sind.

**26.** Vorrichtung nach einem der vorhergehenden Ansprüche, **dadurch gekennzeichnet, dass** die Zusammensetzung mindestens ein zusätzliches fixierendes Polymer enthält, das unter den nichtionischen, kationischen, anionischen oder amphoteren fixierenden Polymeren ausgewählt ist.

**27.** Verfahren zur Haarbehandlung für die Formgebung oder Festigung der Frisur, **dadurch gekennzeichnet, dass** eine Vorrichtung nach einem der Ansprüche 1 bis 26 verwendet wird.

**28.** Verwendung einer Vorrichtung nach einem der Ansprüche 1 bis 26 für die Herstellung eines Lacks oder eines Aerosolsprays.

## Claims

**1.** Aerosol device comprising a container containing a hair composition formed of a fluid and at least one propellant, as well as means for distributing the composition, **characterized in that**:

(i) the composition comprises, in a cosmetically acceptable medium, at least one polycondensate comprising at least one polyurethane and/or polyurea sequence; and
(ii) the device is suitable for giving an initial flow rate of aerosol composition of less than or equal to 0.75 gram per second,

and the device comprises a valve with a 0.33 mm internal restriction orifice, without an additional gas intake orifice

and with a nozzle orifice measuring between 0.33 and 0.51 mm.

2. Device according to Claim 1, **characterized in that** the initial flow rate of aerosol composition is less than or equal to 0.7 gram per second.

3. Device according to either of the preceding claims, **characterized in that** the fluid contains an organic solvent, the weight ratio of the propellant to the organic solvent being greater than or equal to 1.5, and preferably greater than or equal to 1.75.

4. Device according to any one of the preceding claims, **characterized in that** the polycondensate is formed by an arrangement of blocks.

5. Device according to the preceding Claim 4, **characterized in that** the arrangement of blocks is obtained from:

   (1) at least one compound which contains two or more than two active hydrogen atoms per molecule;
   (2) at least one diol or a mixture of diols containing acid radicals or their salts;
   (3) at least one di- or polyisocyanate.

6. Device according to Claim 5, **characterized in that** the compounds (1) are chosen from the group comprising diols, diamines, polyesterols and polyetherols, or a mixture thereof.

7. Device according to Claim 5, **characterized in that** the compound (2) is a 2,2-hydroxymethylcarboxylic acid.

8. Device according to Claim 5, **characterized in that** the compound (3) is chosen from the group comprising hexamethylene diisocyanate, isophorone diisocyanate, toluylene diisocyanate, diphenylmethane 4,4'-diisocyanate, dicyclohexylmethane 4,4'-diisocyanate, methylenedi(p-phenyl) diisocyanate, methylenebis(4-cyclohexyl isocyanate), isophorone diisocyanate, toluene diisocyanate, 1,5-naphthalene diisocyanate, 4,4'-diphenylmethane diisocyanate, 2,2'-dimethyl-4,4'-diphenylmethane diisocyanate, 1,3-phenylene diisocyanate, 1,4-phenylene diisocyanate, mixtures of 2,4- and 2,6-toluene diisocyanate, 2,2'-dichloro-4,4'-diisocyanatodiphenylmethane, 2,4-dibromo-1,5-diisocyanatonaphthalene, butane 1,4-diisocyanate, 1,6-hexane diisocyanate and 1,4-cyclohexane diisocyanate.

9. Device according to Claim 5, **characterized in that** the polycondensate is formed from at least one additional compound having a silicone skeleton and chosen from the group comprising polysiloxanes, polyalkylsiloxanes or polyarylsiloxanes.

10. Device according to Claim 9, **characterized in that** the polyalkylsiloxanes or polyarylsiloxanes are polyethylsiloxanes, polymethylsiloxanes and polyphenylsiloxanes, optionally containing hydrocarbon-based chains grafted onto the silicon atoms.

11. Device according to any one of Claims 1 to 3,
**characterized in that** the polyurethane and/or polyurea sequences of the polymer have a repeating base unit corresponding to the general formula I' below:

$$- X'-B-X'-CO-NH-R-NH-CO- \qquad (I')$$

in which:

- X' represents O and/or NH,
- B is a hydrocarbon-based radical, this radical being substituted or unsubstituted, and
- R is a divalent radical chosen from alkylene radicals of aromatic type, $C_1$ to $C_{20}$ aliphatic radicals or $C_1$ to $C_{20}$ cycloaliphatic radicals, these radicals being substituted or unsubstituted.

12. Device according to Claim 11, **characterized in that** B is a $C_1$ to $C_{30}$ divalent hydrocarbon-based radical.

13. Device according to Claim 11, **characterized**

**in that** the radical R is chosen from the group comprising hexamethylene, 4,4'-biphenylenemethane, 2,4-and/or 2,6-tolylene, 1,5-naphthylene, p-phenylene and methylene-4,4-bis-cyclohexyl radicals and the divalent radical derived from isophorone.

**14.** Device according to any one of Claims 1 to 3,
**characterized in that** the polycondensate has a repeating base unit corresponding to the formula (II'):

$$- X'-P-X'-CO-NH-R-NH-CO- \quad (II')$$

in which:

- P is a polysiloxane segment,
- X' represents O and/or NH, and
- R is a divalent radical chosen from alkylene radicals of aromatic type, $C_1$ to $C_{20}$ aliphatic radicals and $C_1$ to $C_{20}$ cycloaliphatic radicals, these radicals being substituted or unsubstituted.

**15.** Device according to any one of the preceding claims, **characterized in that** the composition comprises, in a relative proportion by weight, between 0.1 and 20% polycondensate.

**16.** Device according to the preceding Claim 15,
**characterized in that** the composition comprises, in a relative proportion by weight, between 1 and 15% polycondensate.

**17.** Device according to the preceding Claim 15 or 16, **characterized in that** the composition comprises, in a relative proportion by weight, between 2 and 8% polycondensate.

**18.** Device according to Claim 3, **characterized in that** the composition comprises an organic solvent which is present in a relative concentration by weight of between 7.5 and 70%.

**19.** Device according to Claim 18, **characterized**
**in that** the composition comprises an organic solvent which is present in a relative concentration by weight of between 10 and 50%.

**20.** Device according to Claim 18 or 19,
**characterized in that** the composition comprises an organic solvent which is present in a relative concentration by weight of between 10 and 25%.

**21.** Device according to any one of the preceding claims, **characterized in that** the propellent gas is present in a relative concentration by weight of between 15 and 70%.

**22.** Device according to any one of the preceding claims, **characterized in that** the propellent gas is present in a relative concentration by weight of between 25 and 60%.

**23.** Device according to either of the preceding Claims 21 and 22, **characterized in that** the propellant gas is present in a relative concentration by weight of between 30 and 50%.

**24.** Device according to any one of the preceding claims, **characterized in that** it comprises a press-button having a turbulent nozzle, the nozzle orifice being between 0.4 and 0.5 mm in size.

**25.** Device according to any one of the preceding claims, **characterized in that** the composition also contains conventional cosmetic additives chosen from the group comprising fatty substances, thickeners, softeners, antifoaming agents, moisturizers, antiperspirants, basifying agents, dyes, pigments, fragrances, preserving agents, surfactants, hydrocarbon-based polymers, silicones, volatile or nonvolatile, in particular anionic silicones, polyols, proteins and vitamins.

**26.** Device according to any one of the preceding claims, **characterized in that** the composition contains at least one additional fixing polymer chosen from the group comprising nonionic, cationic, anionic or amphoteric fixing polymers.

**27.** Hair process for shaping or maintaining the hairstyle, **characterized in that** it comprises the use of a device in accordance with any one of Claims 1 to 26.

**28.** Use of a device according to any one of Claims 1 to 26 for the manufacture of a lacquer or an aerosol spray.